Europäisches Patentamt

European Patent Office

Office européen des brevets

(19)

(11) Veröffentlichungsnummer: **0 307 495 B1**

(12) **EUROPÄISCHE PATENTSCHRIFT**

(45) Veröffentlichungstag der Patentschrift: 24.04.91

(21) Anmeldenummer: 87113536.4

(22) Anmeldetag: 16.09.87

(51) Int. Cl.⁵: **G06F 3/06**, G06F 5/06, A61F 9/00

(54) **Chirurgisches Instrument.**

(43) Veröffentlichungstag der Anmeldung:
**22.03.89 Patentblatt 89/12**

(45) Bekanntmachung des Hinweises auf die
Patenterteilung:
**24.04.91 Patentblatt 91/17**

(84) Benannte Vertragsstaaten:
**CH DE FR GB IT LI NL**

(56) Entgegenhaltungen:
**DE-U- 8 614 218**
**FR-A- 881 346**
**US-A- 4 530 117**

(73) Patentinhaber: **Erbe Elektromedizin GmbH.**
**Waldhörnlestrasse 17**
**W-7400 Tübingen(DE)**

(72) Erfinder: **van Gerven, Johannes Theodores Maria**
**Eugenstrasse 10**
**W-7403 Ammerbuch 1(DE)**

(74) Vertreter: **Endlich, Fritz, Dipl.-Phys.**
**Postfach 1326 Blumenstrasse 8**
**W-8034 Germering(DE)**

## Beschreibung

Die Erfindung betrifft ein chirurgisches Instrument zur Durchführung von intraokularen Operationen. Ein derartiges Instrument ist beispielweise aus der US-A-4530177 bekannt.

In den letzten Jahren wurden weit über eine Million künstlicher Linsen als Intraokularlinsen implantiert. Derartige implantierbare Linsen weisen mehr oder weniger elastische, offene oder geschlossene Bügel auf, die zur Fixation und gleichzeitigen Zentrierung im Äquatorbereich des ausgeschälten Kapselsackes dienen. Dabei hängt die Spannung der Bügel vor allem von der Größe des Umfangbereichs ab, in dem die Bügel von innen an dem Kapselsackäquator anliegen. Von der Art der Fixation hängt nicht nur die bleibende und stabile Zentrierung des optischen Teils im Bereich der Pupille ab, sowie die Sicherung gegen ungewollte Rotation oder ein Verkippen der Linse, sondern auch die erwünschte faltenfreie Ausspannung der hinteren Linsenkapsel. Je gleichmäßiger die Verteilung der Spannung innerhalb des Kapselsackes ist, desto wahrscheinlicher ist es, daß es auch lange Zeit nach der Implanation nicht zur Ausbildung störender Kapselunregelmäßigkeiten kommt. Operationstechnisch ist es deshalb verhältnismäßig schwierig, die Bügel in einer geeigneten Fixierlage zu verankern. Normalerweise findet in diesem Zusammenhang ein Positionierhäkchen Verwendung, das in eines von mehreren Positionierlöchern an der Linse eingehakt wird, um nach dem Einführen der Bügel mit einer Pinzette in den Fixierbereich eine vorteilhafte Fixierlage durch Verdrehen der Linse zu erzielen.

Es ist deshalb Aufgabe der Erfindung, ein bei der Durchführung von intraokularen Operationen verwendbares chirurgisches Instrument anzugeben, dessen Verbindung es einerseits ermöglicht, daß eine genauere Einstellung der Fixierlage möglichst schnell und einfach erzielbar ist, und daß andererseits beim Einführen dieses Instruments und bei dessen Handhabung in der intraokularen Lage möglichst keine traumatischen Gefährdungen verursacht werden.

Diese Aufgabe wird erfindungsgemäß durch ein chirurgisches Instrument zur Durchführung von intraokularen Operationen gelöst, das die kennzeichnenden Merkmale des Patentanspruchs 1 aufweist. Zweckmäßige Ausgestaltungen der Erfindung sind Gegenstand der Unteransprüche.

Ein derartiges Instrument kann durch eine für die Operation erforderliche Schnittöffnung ohne traumatische Gefährdung in die vordere Augenkammer eingeführt und in dieser verschwenkt werden, um in einer geeigneten Schwenklage mit Hilfe des bei der Operation benutzten Mikroskops die Lage des betreffenden Bügels bei dessen Positionierung in eine geeignete Lage beobachten zu können. Dieses Spiegelelement kann einerseits so groß ausgebildet werden, daß durch unterschiedliche Anordnungslagen des Spiegelelements die interessierenden Bereiche im Kapselsack beobachtet werden können. Das Spiegelelement wird andererseits höchstens so groß ausgebildet, daß es mit seiner Umrißlinie beim Verschwenken nicht mit der Innenfläche der Hornhaut in Berührung gelangt.

Durch die Verwendung eines derartigen chirurgischen Instruments ist es deshalb im allgemeinen möglich, eine genauere Fixierlage der Bügel bei verringerter Operationszeit zu erzielen. Eine Verringerung der Operationszeit ist auch deshalb von Bedeutung, weil sich bei einer Operation die Irisöffnung verhältnismäßig schnell verkleinert, wordurch nicht nur das Blickfeld verringert, sondern auch die Beschädigungsgefahr bei der Benutzung eines Positionierhäkchens vergrößert wird.

Anhand der Zeichnung soll die Erfindung beispielsweise näher erläutert werden. Es zeigen:

Fig. 1    eine schematische Schnittansicht durch die Vorderkammer eines Auges, in die eine Kunstlinse eingesetzt ist,

Fig. 2    eine Draufsicht auf eine bekannte Kunstlinse der interessierenden Art,

Fig. 3    eine schematische Seitenansicht eines Ausführungsbeispiels eines chirurgischen Instruments gemäß der Erfindung,

Fig. 4    eine Schnittansicht entsprechend der Linie A-A in Fig.3,

Fig. 5    und 6 zwei unterschiedliche Ausführungsformen des vorderen Endbereichs des Instruments in Fig. 3,

Fig. 7    eine Fig. 1 entsprechende Darstellung nach dem Einführen des vorderen Endes des Instruments in Fig. 3; und

Fig. 8    und Fig. 9 eine Schnittansicht beziehungsweise eine Seitenansicht eines abgewandelten Ausführungsbeispiel des Spiegelelements in Fig. 3.

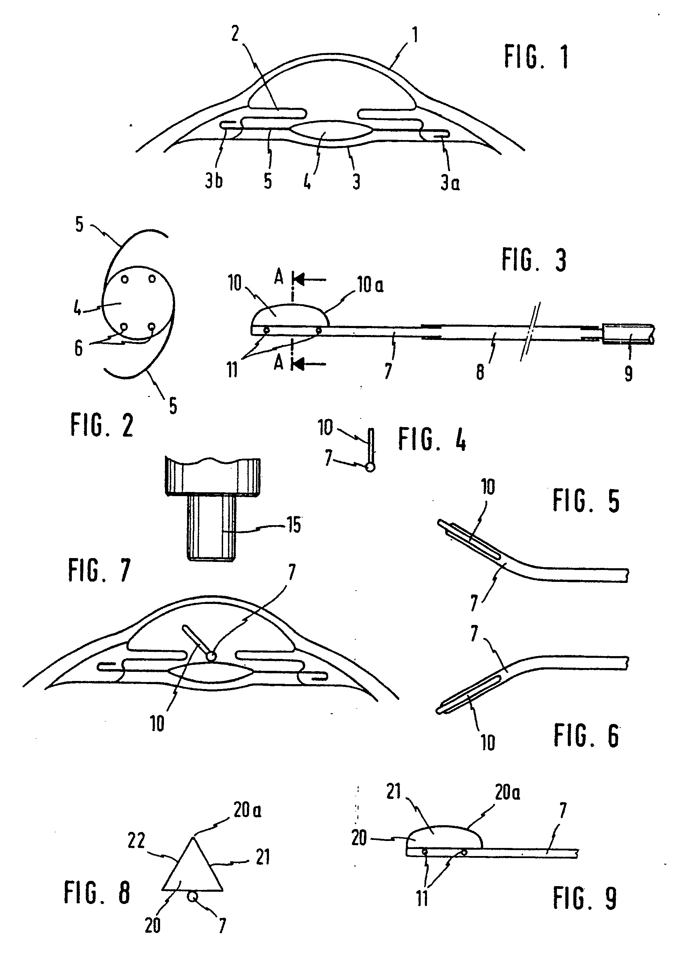

Die in Fig. 1 dargestellte Schnittansicht durch die vordere Augenkammer eines Auges zeigt die Hornhaut 1, die Iris 2 und die hintere Kapselwand 3 des Kapselsackes in schematischer Darstellung. Zwischen der Iris 2 und der Kapselwand 3 ist eine Kunstlinse 4 eingesetzt, von der Fig. 2 eine Draufsicht zeigt. Die Kunstlinse 4 weist zwei elastische Bügel 5 auf, deren Halterungsbereiche 3a und 3b im Äquatorbereich des Kapselsacks in einer geeigneten Lage fixiert werden sollen. Zum Zwecke der Positionierung weist die Linse 4 in Fig. 2 dargestellte Positionierlöcher 6 auf, so daß ein Positionierhäkchen mit einem der Positionierlöcher 6 in Eingriff gebracht werden kann, um die Kunstlinse drehen zu können.

Bei dem in Fig. 3 dargestellten Ausführungsbeispiel eines chirurgischen Instruments gemäß der Erfindung ist ein Infusionsröhrchen 7 vorgesehen, das über einen Infusionsschlauch 8 mit einem Infusionsanschluß 9 in Verbindung steht. Am vorderen Ende des Infusionsröhrchens 7 ist ein Spiegelelement 10 befestigt, das vorzugsweise durch ein Plättchen gebildet ist, dessen beide gegenüberliegende Oberflächen verspiegelt sind.

Das Spiegelelement 10 hat eine von seinem Befestigungsbereich vorgewölbte, der Wölbung der Hornhaut des Auges angepaßte Umrißlinie 10a. Die beiden Spiegelflächen des Spiegelelements 10 werden einerseits so groß gewählt, daß eine zuverlässige Beobachtung in den beiden Halterungsbereichen 3a und 3b in Fig. 1 mit Hilfe eines in Fig. 7 dargestellten Mikroskops 15 möglich ist. Wenn sich das Spiegelelement 10 in der in Fig. 7 dargestellten Lage befindet, kann der rechte Halterungsbereich beobachtet werden, während nach dem Verschwenken des Spiegelelements um 90° im Uhrzeigersinn der linke Halterungsbereich in Fig. 7 beobachtete werden kann.

Das plättchenförmige Spiegelelement 10 hat andererseits nur eine so große Spiegelfläche, daß beim Verschwenken des Spiegelelements noch ein Abstand zwischen dessen Umrißlinie 10a und der Innenfläche der Hornhaut 1 beibehalten werden kann, um eine reibende Berührung mit der Innenfläche der Hornhaut zu vermeiden.

Um ein Zusammenfallen der Vorderkammer während der Operation zu vermeiden, weil Kammerwasser durch die Schnittöffnung während der Operation abfließen kann, wird das Spiegelelement 10 vorzugsweise an dem dargestellten Infusionsröhrchen 7 einer Infusionseinrichtung befestigt, die zur Zuführung von Infusionslösung dient, die durch Infusionsöffnungen 11 austritt. Es ist deshalb ein zusätzliches Instrument nicht unbedingt erforderlich, an dem das in die Augenkammer einzuführende Spiegelement befestigt ist.

Wie in den Fig. 5 und 6 dargestellt ist, kann das vordere Ende des Instruments angrenzend an den Befestigungsbereich des Spiegelelements 10 nach rechts beziehungsweise links abgewinkelt oder abgebogen sein, da der Arzt seitlich von dem Kopf des Patienten sitzt und dessen Nase bei der Handhabung des Geräts im Wege sein kann.

Bei dem in den Fig. 8 und 9 dargestellten abgewandelten Ausführungsbeispiel ist ein prismenförmiges Spiegelelement 20 vorgesehen, das durch ein Dachprisma mit einer gewölbten Firstlinie 20a gebildet ist, und wobei die beiden an die Firstlinie angrenzenden Seitenflächen (21,22) des Dachprismas verspiegelt sind.

Mit Hilfe eines chirurgischen Instruments mit einem derart asugebildeten Spiegelelement ist deshalb die Lage der beispielsweise als Bügel ausgebildeten Fixierelemente der Kunstlinse beobachtbar, wodurch eine optimale Positionierung in der Fixierlage ermöglicht wird, so daß die bisher als unvermeidlich angesehene Anzahl von Komplikationen verringert werden kann, die sich bei einer ungeeigneten Lage der Fixierelemente ergeben konnten.

**Ansprüche**

1. Chirurgisches Instrument zur Durchführung von intraokularen Operationen, **dadurch gekennzeichnet,** daß am vorderen Ende des Instruments ein Spiegelelement (10;20) befestigt ist, das eine von seinem Befestigungsbereich vorgewölbte, der Wölbung der Hornhaut des Auges angepaßte Umrißlinie (10a;20a) aufweist, wobei beim Verschwenken des Spiegelelements mit einer in der Ebene der Iris (2) liegenden Schwenkachse noch ein Abstand zwischen dessen Umrißlinie und der Innenfläche der Hornhaut beibehalten werden kann.

2. Chirurgisches Instrument nach Anspruch 1, **dadurch gekennzeichnet,** daß das Spiegelelement (10) durch ein Plättchen gebildet ist, dessen beide gegenüberliegenden Oberflächen verspiegelt sind.

3. Chirurgisches Instrument nach Anspruch 1, **dadurch gekennzeichnet,** daß das Spiegelelement (20) durch ein Dachprisma mit einer gewölbten Firstlinie (20a) gebildet ist, und daß die beiden an die Firstlinie angrenzenden Seitenflächen des Dachprismas verspiegelt sind.

4. Chirurgisches Instrument nach einem der vorhergehenden Ansprüche, **dadurch gekennzeichnet,** daß das vordere Ende des Instruments ein Infusionsröhrchen (7) ist.

5. Chirurgisches Instrument nach einem der vorhergehenden Ansprüche, **dadurch gekennzeichnet,** daß das vordere Ende des Instruments angrenzend an den Befestigungsbereich des Spiegelelements (10;20) abgewinkelt oder abgebogen ist.

**Claims**

1. A surgical instrument for the performance of intraocular operations, **characterized in that** a mirror element (10;20) is fastened at the front end of the instrument, which mirror element has a contour line (10a;20a) domed forward from its fastening region and adapted to the curvature of the cornea of the eye, whereby during swivelling the mirror element with a swivel axis in the plane of the iris (2), a distance can still be kept between its contour line and the inner surface of the cornea.

2. A surgical instrument according to claim 1, **characterized in that** the mirror element (10) is formed by a small plate, the two opposite surfaces whereof are given a mirror coating.

3. A surgical instrument according to claim 1, **characterized in that** the mirror element (20) is formed by a roof prism with a curved ridge line (20a) and that the two side surfaces of the roof prism adjoining the ridge line are given a mirror coating.

4. A surgical instrument according to one of the preceding claims, **characterized in that** the front end of the instrument is a small infusion tube (7).

5. A surgical instrument according to one of the preceding claims, **characterized in that** the front end of the instrument adjoining the fastening region of the mirror element (10;20) is angled or bent.

**Revendications**

1. Instrument de chirurgie destiné à effectuer des opérations intraoculaires, caractérisé en ce qu'à l'extrémité avant de l'instrument est fixé un élément de miroir (10;20), qui comporte une ligne de contour (10a;20a) pré-incurvée par sa zone de fixation et adaptée à la courbure de la cornée de l'oeil de manière à pouvoir maintenir, lors du basculement de l'élément de miroir, une distance entre sa ligne de contour et la surface intérieure de la cornée, si l'axe de rotation se situe au milieu de l'iris.

2. Instrument de chirurgie suivant la revendication 1, caractérisé en ce que l'élément de miroir (10) est formé d'une plaquettte dont les deux surfaces opposées sont rendues réfléchissantes.

3. Instrument de chirurgie suivant la revendication 1, caractérisé en ce que l'élément de miroir (20) est formé d'un prisme en forme de toit ayant une ligne de faîte (20a) incurvée, et en ce que les deux surfaces latérales du prisme en forme de toit, qui sont adjacentes à la ligne de faîte, sont rendues réfléchissantes.

4. Instrument de chirurgie suivant l'une des revendications précédentes, caractérisé en ce que l'extrémité antérieure de l'instrument est un petit tube infusif (7).

5. Instrument de chirurgie suivant l'une des revendications précédentes, caractérisé en ce que l'extrémité antérieure de l'instrument est coudée ou incurvée dans le voisinage de la région de fixation de l'élément de miroir (10;20).

FIG. 1

FIG. 2

FIG. 3

FIG. 4

FIG. 5

FIG. 6

FIG. 7

FIG. 8

FIG. 9